# EUROPÄISCHE PATENTANMELDUNG

(11) **EP 4 643 891 A2**
(43) Veröffentlichungstag der Anmeldung: **05.11.2025**
(21) Anmeldenummer: 25194800.6
(22) Anmeldetag: 11.02.2020
(51) Int. Cl.: A61L 2/18

(54) **VERFAHREN ZUR VERMEIDUNG DES MIKROBIELLEN BEFALLS EINER REINIGUNGSVORRICHTUNG FÜR EINE DOSIERANLAGE UND REINIGUNGSVORRICHTUNG**

(30) Priorität: 11.02.2019 DE 102019103273
(62) Teilanmeldung aus: 20705898.3
(71) Anmelder: Brillux GmbH & Co. KG, 48163 Münster (DE)
(72) Erfinder: HÖRSTING, Ingo, 48317 Drensteinfurt (DE); ERBER, Dietmar, 48143 Münster (DE); STORB, Rainer, 48301 Nottuln (DE); FELS, Sven, 48153 Münster (DE)
(74) Vertreter: Cohausz & Florack

(57) **Zusammenfassung**

Dargestellt und beschrieben wird ein Verfahren zur Vermeidung des mikrobiellen Befalls einer Reinigungsvorrichtung (X2), insbesondere einer Reinigungsvorrichtung (X2) für eine Dosieranlage, wobei die Reinigungsvorrichtung (X2) wenigstens ein mechanisches Reinigungselement (X29) mit wenigstens einer Reinigungsfläche (X31) aufweist, wobei das Verfahren dadurch gekennzeichnet ist, dass die wenigstens eine Reinigungsfläche (X31) des wenigstens einen mechanischen Reinigungselements (X29) zumindest zeitweise mit einem Oxidationsmittel beaufschlagt wird. Ferner betrifft die vorliegende Erfindung ein Verfahren zum Reinigen einer Dosieranlage, insbesondere für eine Dispersion, speziell einer Farbdosieranlage, eine Reinigungsvorrichtung und eine Dosieranlage.

## Beschreibung

Die Erfindung betrifft ein Verfahren zur Vermeidung des mikrobiellen Befalls einer Reinigungsvorrichtung, insbesondere einer Reinigungsvorrichtung für eine Dosieranlage, wobei die Reinigungsvorrichtung wenigstens ein mechanisches Reinigungselement mit wenigstens einer Reinigungsfläche aufweist. Ferner betrifft die Erfindung ein Verfahren zum Reinigen einer Dosieranlage, eine Reinigungsvorrichtung sowie eine Dosieranlage.

Dosieranlagen ermöglichen die präzise Bereitstellung einer bestimmten Menge einer Dispersion auf Abfrage. Im Sinne der vorliegenden Erfindung sind mit Dosieranlagen grundsätzlich sämtliche Systeme gemeint, die ein dosiertes Bereitstellen von einem Material ermöglichen. Dosieranlagen kommen insbesondere beim Abmischen von Dispersionsfarben zur Anwendung.

Eine Dosieranlage für Farben ist beispielsweise in der DE 196 54 829 A1 beschrieben. Als besonderes Problem bei Dosieranlagen für Dispersionen erweist sich deren mikrobieller Befall durch Pilze und Bakterien. Dies ist auf eine Lagerung der Dispersion(en) in einem entsprechenden Behälter in der Dosieranlage zum Teil über mehrere Wochen und Monate zurückzuführen. Der mikrobielle Befall ist dabei nicht auf den Lagerungsbehälter in der Dosieranlage beschränkt, sondern verteilt sich auf sämtliche Elemente der Dosieranlage, die mit der befallenen Dispersion in Kontakt kommen, insbesondere auch auf die Dosiereinheit, mittels derer die gelagerte Dispersion in kontrollierter Menge abgegeben werden kann. Die Dosiereinheit umfasst üblicherweise einen sogenannten Pumpenkopf, über welchen die Dispersion aus der Dosieranlage abgegeben werden kann. Mit der Zeit setzen sich der Pumpenkopf und insbesondere dessen Auslassöffnung bzw. -düse mit Dispersionsresten zu, so dass eine regelmäßige, in der Regel mechanisch durchgeführte Reinigung dieser Auslassöffnung notwendig ist. Hierzu sind aus der Praxis Reinigungsvorrichtungen bekannt, welche beispielsweise eine rotierende Bürste umfassen, mithilfe derer die Reinigung der Auslassöffnung zuverlässig erfolgt. Die rotierende Bürste ihrerseits wird im Betrieb der Reinigungsvorrichtung in einem Reinigungsbad gereinigt bzw. gespült, in welches sie im Reinigungsbetrieb teilweise eingetaucht ist. Wird das Reinigungsbad nicht regelmäßig gewechselt, so können sich Pilze und Bakterien bilden, welche die Reinigungsvorrichtung kontaminieren. Weist die Dosieranlage eine Mehrzahl von Lagerungsbehältern für Dispersionen mit dementsprechend einer Mehrzahl von Pumpenköpfen auf, wie dies beispielsweise bei Farbmischanlagen der Fall ist, so kann sich der mikrobielle Befall einer gelagerten Dispersion auch auf die anderen Dispersionen ausbreiten. Dies kann dadurch erfolgen, dass die befallenen Dispersionsrückstände an der Auslassöffnung oder -düse eines Pumpenkopfes abgereinigt werden und sich sodann in dem Reinigungsbad sammeln. Eine Verteilung des mikrobiellen Befalls auf die Pumpenköpfe der zunächst nicht befallenen Dispersionen erfolgt sodann über die Reinigungsbürste, die ihrerseits permanent im Reinigungsbad gespült wird.

Der vorliegenden Erfindung liegt daher die Aufgabe zugrunde, ein Verfahren anzugeben, mithilfe dessen der mikrobielle Befall einer Reinigungsvorrichtung, insbesondere der mikrobielle Befall einer Reinigungsvorrichtung für eine Dosieranlage, wirksam vermieden wird.

Eine weitere Aufgabe der vorliegenden Erfindung besteht ferner darin, eine Reinigungsvorrichtung für eine Dosieranlage, insbesondere für eine Dispersion, speziell für eine Farbdosieranlage, anzugeben, bei der ein mikrobieller Befall der Reinigungsvorrichtung sowie der Dosieranlage wirksam vermieden wird.

Weitere Aufgaben ergeben sich aus den nachfolgenden Ausführungen.

Die vorstehenden Aufgaben werden verfahrensmäßig mit einem Verfahren gemäß dem Oberbegriff des Punktes 1 dadurch gelöst, dass die wenigstens eine Reinigungsfläche des wenigstens einen mechanischen Reinigungselements zumindest zeitweise mit einem Oxidationsmittel beaufschlagt wird.

Der besondere Vorteil des erfindungsgemäßen Verfahrens besteht darin, dass durch Beaufschlagung des wenigstens einen mechanischen Reinigungselements mit einem Oxidationsmittel eine Anlagerung und eine folgende Vermehrung von mikrobiellem Befall, also beispielsweise von Bakterien, Schimmel oder Hefepilzen, wirksam über einen langen Zeitraum verhindert wird. Bei bereits eingesetztem mikrobiellem Befall erlaubt das erfindungsgemäße Verfahren, diesen sicher zu eliminieren.

Überraschend hat sich gezeigt, dass mit dem erfindungsgemäß Verfahren zur Vermeidung des mikrobiellen Befalls einer Reinigungsvorrichtung in einer Dosieranlage, speziell einer Farbdosieranlage, dort gelagerte Farbdispersionen über Monate hinweg vor mikrobiellen Befall geschützt werden können, da einerseits, wie erwähnt, die Anlagerung und eine folgende Vermehrung von mikrobiellem Befall in der Reinigungsvorrichtung selbst wirksam verhindert wird und damit zusammenhängend auch eine Verbreitung des mikrobiellen Befalls, soweit er bereits besteht, in den mit der Reinigungsvorrichtung zu reinigenden Komponenten verhindert wird. Dies gelingt erfindungsgemäß durch das konsequente Beaufschlagen des oder der mechanischen Reinigungselemente der Reinigungsvorrichtung mit dem Oxidationsmittel. Eine in der Dosieranlage gelagerte Dispersion kann hierbei frei von Konservierungsmitteln sein und weist trotzdem einen langanhaltenden Schutz gegen mikrobiellen Befall auf. Selbst bei häufiger Benutzung der Dosieranlage und einem wiederholten Auffüllen des Behälters mit frischer Dispersion wird bei regelmäßiger Reinigung ein Schutz vor Pilz- und Bakterienbefall durch das erfindungsgemäße Verfahren gewährleistet.

Die Beaufschlagung der wenigstens einen Reinigungsfläche des wenigstens einen Reinigungselements der Reinigungsvorrichtung kann kontinuierlich oder diskontinuierlich, beispielsweise in periodischen Zeitabständen (z.B. alle 6, 12 oder 24 Stunden) erfolgen. Dies bemisst sich u.a. an den Umgebungsbedingungen (Temperatur, Luftfeuchtigkeit, usw.), in denen die Reinigungsvorrichtung zum Einsatz kommt und die eine Entstehung oder Vermehrung mikrobiellen Befalls ggf. begünstigen.

Grundsätzlich eignen sich ganz unterschiedliche Oxidationsmittel zur Verhinderung oder Eliminierung des mikrobiellen Befalls. Es kommen Oxidationsmittel infrage, die gasförmig, flüssig oder fest sind. Flüssige und feste Oxidationsmittel werden bevorzugt als wässrige Lösungen eingesetzt, da hierdurch die Handhabung vereinfacht wird. Bevorzugt ist das Oxidationsmittel somit ein Oxidationsfluid. Als besondfers geeignet haben auch gasförmige Oxidationsfluide erwiesen.

Um eine möglichst gründliche und vollständige Beaufschlagung der wenigstens einen Reinigungsfläche des wenigstens einen Reinigungselements zu erreichen, ist nach einer vorteilhaften Ausgestaltung der Erfindung vorgesehen, dass das Oxidationsmittel auf die wenigstens eine mechanische Reinigungsfläche aufgesprüht oder aufgeblasen wird. Darüber hinausgehend ist es bevorzugt, dass das Aufsprühen oder Aufblasen des Oxidationsmittels auf die wenigstens eine mechanische Reinigungsfläche im Wesentlichen entlang ihrer Längserstreckung, bevorzugt vollflächig erfolgt. Hierdurch ist sichergestellt, dass es bei der Beaufschlagung keine unbehandelten Flächenabschnitte gibt, an denen der mikrobielle Befall einsetzen kann.

Hinsichtlich der chemischen Zusammensetzung steht eine große Bandbreite von Oxidationsmitteln zur Verfügung. Vorteilhafterweise ist das Oxidationsmittel ein sauerstoff- oder chlorbasiertes Oxidationsmittel oder eine Mischung davon, bevorzugt ein sauerstoff-basiertes Oxidationsmittel. Vorzugsweise wird das Oxidationsmittel ausgewählt aus der Gruppe bestehend aus Natriumhypochlorit, Kaliumhypochlorit, Javelwasser, Chlor, Ozon, Wasserstoffperoxid, Peroxyessigsäure, Perborat, Percarbonat, und Mischungen davon. Diese Oxidationsmittel stellen starke Oxidationsmittel dar, die das Wachstum von mikrobiellen Organismen effektiv unterbinden.

Besonders bevorzugt wird das Oxidationsmittel ausgewählt aus der Gruppe bestehend aus Natriumhypochlorit, Wasserstoffperoxid, Ozon, und Mischungen davon. Die Oxidationsmittel aus der genannten Gruppe haben sich als besonders gut geeignet für den Schutz der Dispersion vor mikrobiellem Befall herausgestellt, da sie effektive Oxidationsmittel darstellen. Ferner sind diese Oxidationsmittel insbesondere bei den zweckmäßigerweise vorgesehenen Mengen für die Dispersion im Wesentlichen unproblematisch. Insbesondere sorgen die genannten Oxidationsmittel bei den zweckmäßigerweise vorgesehenen Mengen nicht zu einer nennenswerten Veränderung der Farbe und/oder Qualität der Dispersion.

Wie erwähnt, wird der Einsatz eines Oxidationsfluids, d.h. eines gasförmigen oder flüssigen Oxidationsmittels, bevorzugt. Als besonders geeignet erweist sich ein gasförmiges Oxidationsmittel. Gemäß einer besonders bevorzugten Ausführungsform der Erfindung enthält das Oxidationsmittel Ozon. Enthält das Oxidationsmittel Ozon oder wird Ozon als Oxidationsmittel eingesetzt, erfolgt eine besonders langanhaltende antimikrobielle Wirkung. Wird die Reinigungsvorrichtung im Zusammenhang mit einer Dosieranlage für Dispersionen eingesetzt, erweist sich als Vorteil, dass Ozon mit den gängigen Bestandteilen von Dispersionen, insbesondere den gängigen Bestandteilen von Dispersionsfarben oder Pigmentpasten, kompatibel ist. Zudem lässt sich Ozon sehr leicht am Ort der Verwendung herstellen.

Ozon kann beispielsweise aus einer chemischen Reaktion von Kaliumpermanganat mit konzentrierter Schwefelsäure erzeugt werden oder durch Elektrolyse von verdünnter Schwefelsäure, insbesondere bei tiefen Temperaturen. Ozon kann auch aus Luft oder Sauerstoff unter Einwirkung von UV-Strahlung erzeugt werden.

Gemäß einer bevorzugten Ausführungsform des erfindungsgemäßen Verfahrens wird das Ozon in einem Ozongenerator umfassend eine Spannungsquelle, insbesondere einen Hochspannungsgenerator, und eine Entladungseinheit ausgehend von Luft, insbesondere getrockneter Luft, Sauerstoff oder einem Sauerstoffgemisch mit Argon oder Kohlenstoffdioxid erzeugt. Der Aufbau gängiger Ozongeneratoren ist aus dem Stand der Technik bekannt. Gemäß einer gängigen Variante der Ozonherstellung werden in einer Entladungseinheit Sauerstoffmoleküle vorzugsweise durch stille elektrische Entladung, sogenannte "*Koronaentladung*", zu Sauerstoffatomen dissoziiert, wonach noch im Plasma der Entladungsfilamente die Ozonsynthese und Ozonanreicherung stattfindet. Dabei kann der entstehende Ozonanteil an der Endkonzentration des Gasgemisches bei Luft als Ausgangsgas 1 bis 5 Gew.% und bei Sauerstoff als Ausgangsgas 6 bis 13 Gew.% betragen. Vorzugsweise wird Luft als Ausgangsgas zur Ozonerzeugung eingesetzt. Bei der Erzeugung des Ozons in einem Generator wird somit ausgehend von günstigen Startmaterialien auf saubere Weise Ozon hergestellt.

Nach einer besonders vorteilhaften Ausgestaltung der Erfindung wird die wenigstens eine Reinigungsfläche des wenigstens einen mechanischen Reinigungselements im Betrieb der Reinigungsvorrichtung zumindest zeitweise in einem Reinigungsbad gereinigt, wobei das Oxidationsmittel optional auch das Reinigungsbad zumindest zeitweise beaufschlagt.

Bei dieser Ausgestaltung der Reinigungsvorrichtung wird die wenigstens eine Reinigungsfläche des wenigstens einen mechanischen Reinigungselements selbst gereinigt, so dass die an der wenigstens einen Reinigungsfläche anhaftenden mikrobiellen Verunreinigungen von der Reinigungsfläche abgewaschen werden. Hierdurch kann die Reinigungsvorrichtung über einen längeren Zeitraum völlig autark operieren. Da der mikrobielle Befall somit von der Reinigungsfläche in das Reinigungsbad gelangt, kann es sinnvoll sein, dass neben der Reinigungsfläche auch das Reinigungsbad selbst mit dem Oxidationsmittel zumindest zeitweise beaufschlagt wird. In diesem Fall kann also der mikrobielle Befall noch wirksamer bekämpft werden, da Rückstände mikrobiellen Befalls im Reinigungsbad direkt bekämpft werden.

Nach einer weiteren vorteilhaften Ausgestaltung der Erfindung erfolgt das Aufsprühen oder Aufblasen des Oxidationsmittels auf die wenigstens eine Reinigungsfläche des wenigstens einen mechanischen Reinigungselements außerhalb des Reinigungsbads. Hierdurch kann das Oxidationsmittel noch gezielter auf die wenigstens eine Reinigungsfläche geleitet werden.

Das mechanische Reinigungselement der Reinigungsvorrichtung weist erfindungsgemäß wenigstens eine Reinigungsfläche auf. Diese kann unterschiedlich gestaltet sein. Bevorzugt wird eine umlaufende Reinigungsfläche, insbesondere bei zylindrischer Ausgestaltung des mechanischen Reinigungselements. Es versteht sich, dass anstelle einer zylindrischen Auslegung mit kreisförmiger Grundfläche auch elliptische oder mehreckige Grundflächen möglich sind. Anstelle einer umlaufenden Reinigungsfläche sind ferner auch mehrere nicht miteinander zusammenhängende Reinigungsflächen möglich, beispielsweise eine Mehrzahl im Wesentlichen ebener Reinigungsflächen, welche beispielsweise radial nach außen weisend, um eine gemeinsame Achse drehend angeordnet sein können.

Bei der Ausgestaltung der wenigstens einen Reinigungsfläche ist zu berücksichtigen, dass grundsätzlich sämtliche mechanische Reinigungstechniken im Rahmen der vorliegenden Erfindung anwendbar sind, wie beispielsweise Wischen, Schrubben, Schleifen usw.. Orientiert an der jeweils gewählten Reinigungstechnik ist die Reinigungsfläche entsprechend gestaltet, beispielsweise als Bürste oder Schwamm. Nach einer vorteilhaften Ausgestaltung der Erfindung ist das wenigstens eine Reinigungselement als Bürste und seine Reinigungsfläche entsprechend als Bürstenfläche ausgestaltet. Besonders bevorzugt ist dabei die Ausgestaltung als zylindrische oder walzenförmige Bürste. Unabhängig von der geometrischen Gestaltung der Bürstenfläche wird das Oxidationsmittel bevorzugt im Wesentlichen parallel zu den Borsten in die Bürste eingesprüht oder eingeblasen, so dass die Borsten entlang ihrer gesamten Länge von mikrobiellem Befall gereinigt werden können, was zu einem besonders gründlichen Reinigungsergebnis führt.

Ist das wenigstens eine mechanische Reinigungselement als zylindrische oder walzenförmig rotierende Bürste ausgelegt und kommt ferner ein Reinigungsbad für das Reinigungselement zum Einsatz, so kann die Bürste im Betrieb der Reinigungsvorrichtung teilweise in das Bad eingetaucht sein mit der Folge, dass die Bürste während der Reinigung selbst ständig im Reinigungsbad gereinigt bzw. gespült wird, was einen hohen Automatisierungsgrad ermöglicht.

In Bezug auf die konstruktive Ausgestaltung der Zuführung des Oxidationsmittels besteht eine bevorzugte Lösung darin, dass das Oxidationsmittel über wenigstens eine Zuführleitung zugeführt wird, wobei die wenigstens eine Zuführleitung im Wesentlichen entlang der Längserstreckung der wenigstens einen Reinigungsfläche des wenigstens einen mechanischen Reinigungselements angeordnet ist. Dabei weist die wenigstens eine Zuführleitung ferner eine, bevorzugt eine Mehrzahl, von auf die wenigstens eine Reinigungsfläche ausgerichtete Auslassöffnungen zum Aufsprühen oder Aufblasen des Oxidationsmittels auf. Diese konstruktive Lösung zeichnet sich durch einen einfachen und schnell zu realisierenden Aufbau aus und ermöglicht, dass die wenigstens eine Reinigungsfläche des wenigstens einen mechanischen Reinigungselements möglichst vollflächig mit Oxidationsmittel beaufschlagt wird. Insbesondere eignet sich diese konstruktive Lösung für den Einsatz zylindrischer oder walzenförmiger mechanischer Reinigungselemente, wie zylindrischer oder walzenförmiger Schwämme oder Bürsten.

Ein weiterer Aspekt der vorliegenden Erfindung betrifft ein Verfahren zum Reinigen einer Dosieranlage, insbesondere für eine Dispersion, speziell einer Farbdosieranlage, wobei die Dosieranlage wenigstens eine Dosiereinheit mit einem Pumpenkopf aufweist, wobei der Pumpenkopf mittels einer Reinigungsvorrichtung mit wenigstens einem wenigstens eine Reinigungsfläche aufweisenden mechanischen Reinigungselement gereinigt wird, wobei ein mikrobieller Befall der Reinigungsvorrichtung mit einem Verfahren nach einem der Punkte 1 bis 13 vermieden wird.

Für die Vorteile dieses Reinigungsverfahrens gelten die vorstehend erläuterten Vorteile entsprechend. Insbesondere kommen die Vorteile beim Einsatz des Reinigungsverfahrens bei einer Farbdosieranlage mit einer Mehrzahl von Dosiereinheiten zu Tragen, da hier durch das erfindungsgemäße Verfahren die Ausbreitung eines mikrobiellen Befalls von einer Dosiereinheit auf mehrere oder alle anderen Dosiereinheiten wirksam vermieden werden kann, was sich in den aus der Praxis bekannten Reinigungsverfahren stets als besonderes Problem darstellte.

Vorrichtungsmäßig wird die eingangs gestellte Aufgabe mit einer Reinigungsvorrichtung für eine Dosieranlage, insbesondere für eine Dispersion, speziell für eine Farbdosieranlage, umfassend wenigstens ein mechanisches Reinigungselement mit wenigstens einer Reinigungsfläche sowie eine Zuführeinheit für ein Oxidationsmittel zur zumindest zeitweisen Beaufschlagung der wenigstens einen Reinigungsfläche mit einem Oxidationsmittel gelöst, wobei die Reinigungsvorrichtung zum Reinigen der Dosieranlage gemäß Punkt 14 konfiguriert ist.

Wiederum gilt für die Vorteile der erfindungsgemäßen Vorrichtung das vorstehend Gesagte entsprechend.

Nach einer vorteilhaften Ausgestaltung der Reinigungsvorrichtung umfasst die Reinigungsvorrichtung ein Reinigungsbad zum zumindest zeitweisen Reinigen der wenigstens einen Reinigungsfläche des wenigstens einen mechanischen Reinigungselements im Betrieb der Reinigungsvorrichtung.

Ein weiterer Aspekt der vorliegenden Erfindung betrifft eine Dosieranlage für eine Dispersion, insbesondere Farbdosieranlage, umfassend eine Reinigungsvorrichtung nach Punkt 15 oder 16.

Die erfindungsgemäße Dosieranlage kann neben der erfindungsgemäßen, mit Oxidationsmittel beaufschlagbaren Reinigungsvorrichtung auch noch Vorrichtungen zur Vermeidung des mikrobiellen Befalls anderer Teile der Dosieranlage, insbesondere der in dem/den Behälter(n) befindlichen Dispersion(en), umfassen. Insbesondere kann die Dosieranlage eine Vorrichtung umfassen, mit welcher ein Oxidationsmittel wie z.B. Ozon in einen oder mehrere der Behälter, in denen die Dispersion gelagert ist, eingebracht wird, wie in der internationalen Patentanmeldung PCT/EP2019/052537 beschrieben (vgl. insbesondere Fig. 1 und die dazugehörige Figurenbeschreibung der PCT/EP2019/052537). Das Einbringen von Oxidationsmittel, insbesondere von Ozon, in den/die Behälter der Dosieranlage kann beispielsweise mit Hilfe von geeigneten Adaptern erfolgen, wie in der internationalen Patentanmeldung PCT/EP2019/052537 beschrieben (vgl. insbesondere Fig. 3, 5, 6, 7a, 7b, 8a und 8b sowie die dazugehörigen Figurenbeschreibungen der PCT/EP2019/052537). Auf Offenbarung in der internationalen Patentanmeldung PCT/EP2019/052537 wird in diesem Zusammenhang ausdrücklich Bezug genommen. Ein zumindest zeitweises Beaufschlagen der Reinigungsfläche des wenigstens einen mechanischen Reinigungselements der Reinigungsvorrichtung, wie hierin beschrieben, kombiniert mit einem Einbringen von Oxidationsmittel in einen oder mehrere der Behälter, in denen die Dispersion gelagert ist, wie in der PCT/EP2019/052537 beschrieben, stellt eine besonders bevorzugte Ausführungsform der vorliegenden Erfindung dar.

Im Folgenden wir die vorliegende Erfindung anhand einer ein Ausführungsbeispiel der Erfindung darstellenden Zeichnung erläutert. Es zeigen:
- Fig. 1A: eine Dosieranlage für eine Dispersionsfarbe mit einer Reinigungsvorrichtung in schematisierter Draufsicht,
- Fig. 1B: die Dosieranlage der Fig. 1A in schematisierter Seitenansicht,
- Fig. 2: die Reinigungsvorrichtung der Dosieranlage der Fig. 1 in perspektivischer Ansicht,
- Fig. 3: die Reinigungsvorrichtung der Fig. 2 im Querschnitt gemäß Schnittlinie III-III aus Fig. 2,
- Fig. 4A: die Zuführung eines Oxidationsmittels der Reinigungsvorrichtung der Fig. 2 in vergrößerter perspektivischer Ansicht und
- Fig. 4B: die Zuführung eines Oxidationsmittels der Reinigungsvorrichtung der Fig. 2 in zu Fig. 4A gedrehter perspektivischer Ansicht.

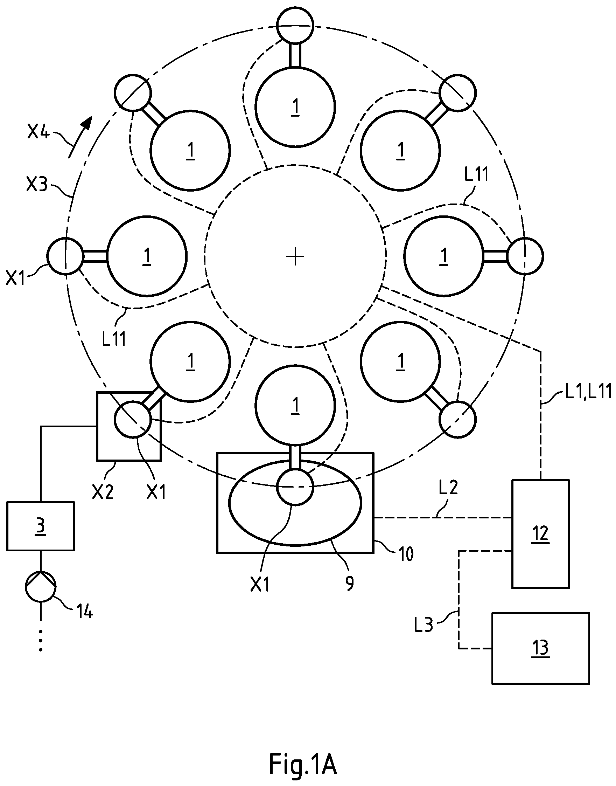

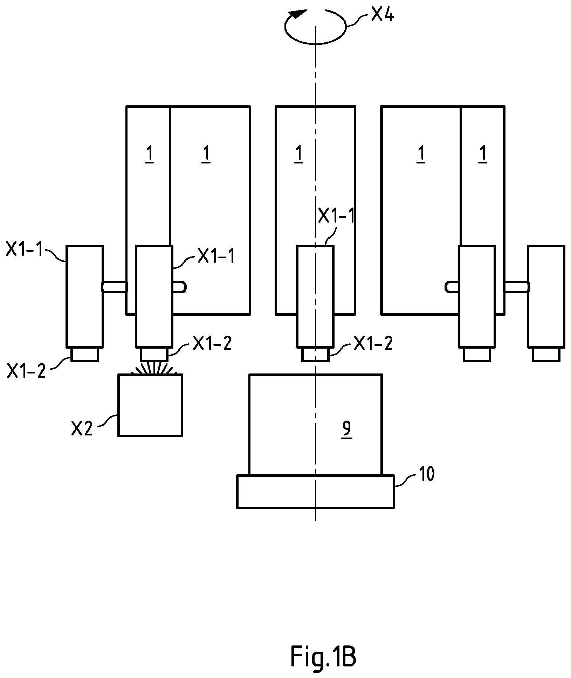

Fig. 1 zeigt eine bevorzugte Ausführungsform einer Dosieranlage zum Mischen einer Dispersionsfarbe mit einer Reinigungsvorrichtung in schematisierter Draufsicht. Es versteht sich, dass prinzipiell der gleiche Aufbau auch zur Dosierung und/oder Mischung anderer Materialien genutzt werden kann.

Die Dosieranlage der Fig. 1 umfasst im Einzelnen eine Mehrzahl vorliegend karussellartig auf einem mit strichpunktierter Linie dargestellten Teller X4 angeordneter Behälter 1, wobei jeder Behälter 1 über eine Zuleitung mit einer Dosiereinheit X1 verbunden ist. Über eine Steuerleitung L1 ist der Antrieb des Tellers X4 mit einem Steuerungsrechner 12 verbunden. Ferner sind die Dosiereinheiten X1 jeweils über Steuerleitungen L11 mit dem Steuerungsrechner verbunden. Wie in der Draufsicht der Fig. 1 erkennbar, ist die Dosiereinheit X1 eines der Farbbehälter 1 über einem Aufnahmebehältnis, vorliegend einem Eimer 9, angeordnet, so dass die in diesem Behälter 1 gelagerte Dispersion über die zugehörige Dosiereinheit X1 in den Eimer 9 dosiert werden kann. Der Eimer 9 seinerseits steht auf einer Waage 10, die über eine Steuerleitung L2 mit dem Steuerungsrechner 12 verbunden ist. Der Steuerungsrechner 12 wiederum ist über eine Steuerleitung L3 mit einem Etikettendrucker 13 verbunden.

Die bezogen auf die über dem Eimer 9 positionierte Dosiereinheit X1 im Uhrzeigersinn X5 benachbarte Dosiereinheit X1 ist oberhalb einer Reinigungsvorrichtung X2 derart positioniert, dass die in den Fig. 1B bis 4B noch im Detail beschriebene Reinigungsvorrichtung X2 den Pumpenkopf X6 (vgl. Fig. 1B) der Dosiereinheit X1 reinigen kann. Wie im Folgenden noch näher erläutert wird, umfasst die Reinigungsvorrichtung X2 ein mechanisches Reinigungselement in Form einer rotierenden zylindrischen Bürste X29 mit einer als Bürstenfläche ausgebildeten zylindrischen Reinigungsfläche X31, mithilfe derer der Pumpenkopf X6 einer jeden Dosiereinheit X1 von Farbrückständen gereinigt werden kann.

Wie in Fig. 1A ebenfalls schematisch dargestellt, ist die Reinigungsvorrichtung X2 über eine Zuführleitung mit einem Ozongenerator 3 verbunden, in den wiederum über eine Membranpumpe 14 Luft oder Sauerstoff eingeleitet wird. Im Ozongenerator 3, wird beispielsweise mittels einer Koronaentladung Ozon erzeugt, welches über die Zuführleitung X24 (in Fig. 1A nur schematisch dargestellt) in die Reinigungsvorrichtung X2 zwecks Beaufschlagung der rotierenden Bürste X29 eingeleitet wird.

Im Betrieb der Dosieranlage berechnet der Steuerungsrechner 12 nach kundenseitiger Eingabe eines gewünschten Farbtons und des gewünschten Volumens die Anteile der in den Behältern 1 gespeicherten Grundfarben und steuert den Antrieb des Tellers X4 derart an, dass nacheinander die Dosiereinheiten X1 der Behälter 1 mit den erforderlichen Grundfarben über dem Eimer 9 positioniert werden und die berechnete Menge in den Eimer 9 abgegeben wird, um so den gewünschten Farbton im Eimer 9 zu erhalten. Dies wird mittels der mit dem Steuerungsrechner über die Leitung L2 verbundenen Waage 10 überwacht. Der Antrieb des Tellers X4 wird derart angesteuert, dass er sich stets im UhrzeigersinnX5 dreht. Dies führt dazu, dass der Pumpenkopf X6 einer jeden am Farbmischprozess beteiligten Dosiereinheit X1 unmittelbar nach Einsatz durch die Reinigungsvorrichtung X2 gereinigt und somit von Dispersionsrückständen befreit wird. Über den Etikettendrucker 13 wird das passende Etikett für die Farbmischung gedruckt.

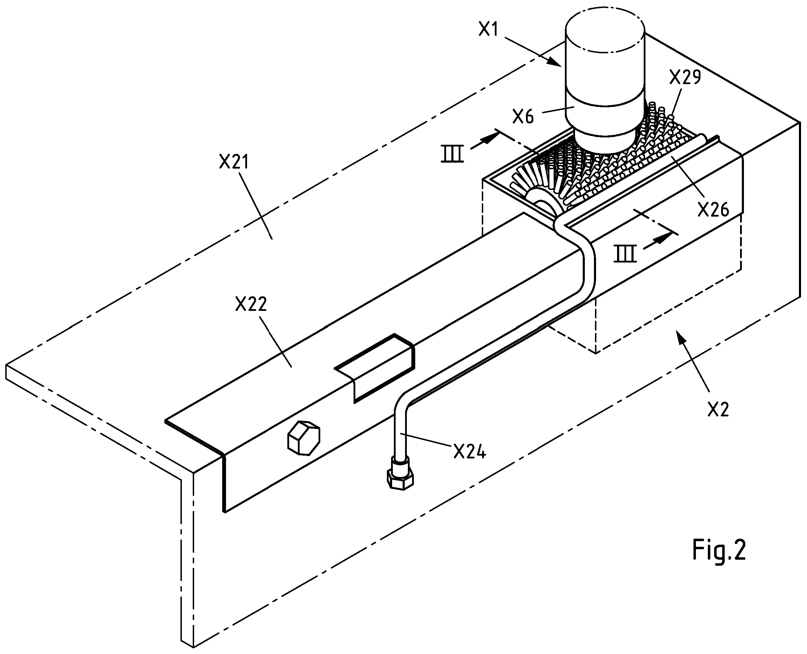

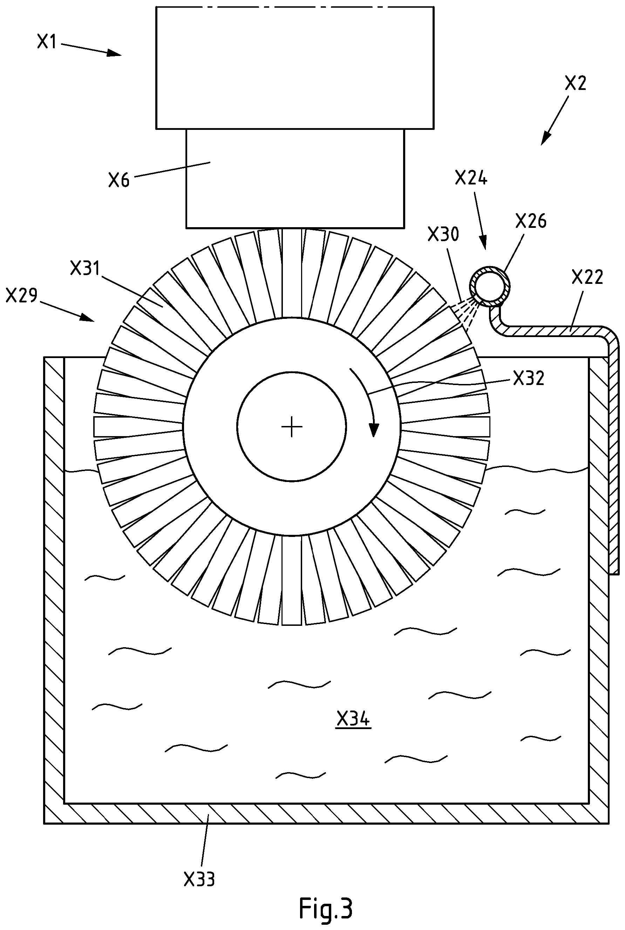

Fig. 2 und 3 zeigen die Reinigungsvorrichtung X2 der Dosieranlage der Fig. 1 in perspektivischer Ansicht sowie im Querschnitt gemäß Schnittlinie III-III aus Fig. 2.

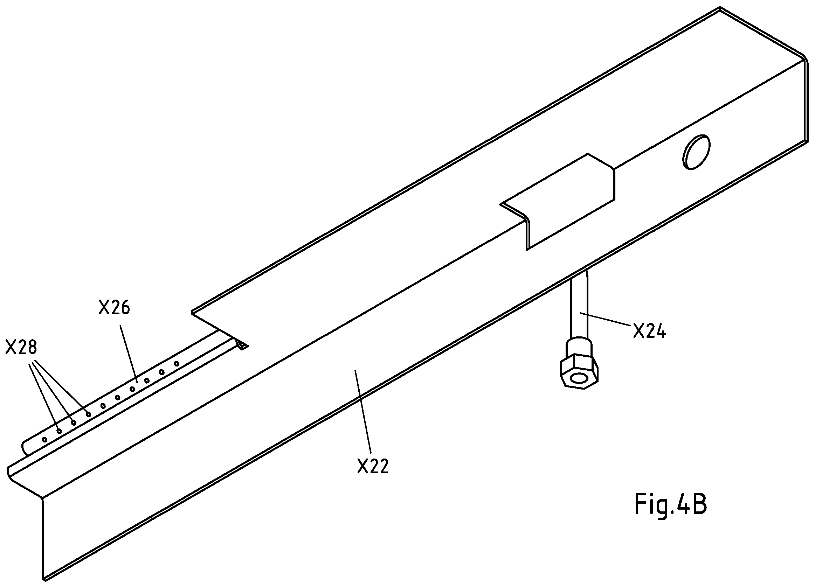

Gemäß Fig. 3 umfasst die Reinigungsvorrichtung X2 als zentrales Element eine in der vorliegenden Ansicht im Uhrzeigersinn X32 rotierende Bürste X31, die im Betrieb mit den oberseitig angeordneten Borsten der Bürstenfläche X31 den Pumpenkopf X6 von Dispersionsrückständen reinigt, während die unterseitigen in ein Reinigungsbad X34 eingetauchten Borsten der Bürste X29 in dem Reinigungsbad X34 von den Farbrückständen befreit werden. Das Reinigungsbad X34 wird vorliegend durch ein Wasserbad gebildet, welches sich in einem Behälter X33 befindet. Wie insbesondere in Fig. 2 erkennbar ist, wird über die Zuführleitung X24 ein Ozon enthaltender Gasstrom als Oxidationsmittel über eine Mehrzahl von in einem Austrittsbereich X26 der Zuführleitung angeordnete Austrittsöffnungen X28 (vgl. Fig. 4B) auf die Bürste X29 geblasen, wobei der Gasstrom im Wesentlichen parallel zu den Borsten der Bürstenfläche X31, d.h. im Wesentlichen radial in die Bürste eingeblasen wird. Der Behälter X33 des Reinigungsbades X34 ist vorliegend über ein Befestigungsprofil X22 mit einem Gehäuse X21 verbunden.

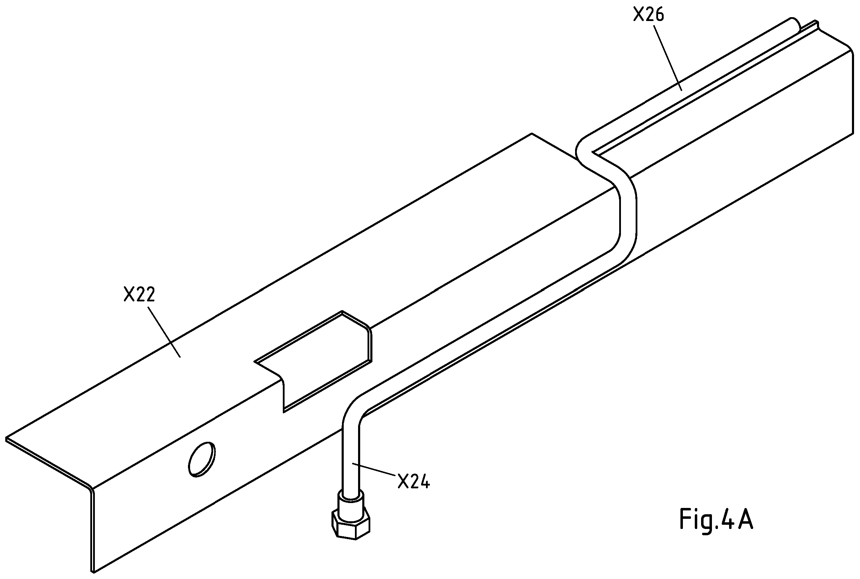

Die Befestigungsschiene X22 samt Zuführleitung X24 für den Ozon enthaltenden Gasstrom ist in den Fig. 4A und 4B nochmals in zweifacher Ansicht perspektivisch dargestellt, wobei in der Darstellung der Fig. 4B die Austrittsöffnungen X28 für den Gasstrom im parallel zur Achse der Bürste X29 ausgerichteten Austrittsbereich X26 der Zuführleitung X24 deutlich erkennbar sind.

Der besondere Vorteil der Reinigungsvorrichtung sowie des Reinigungsverfahrens besteht darin, dass weder im Reinigungsbad X34 noch in der Bürste X29 die Gefahr des mikrobiellen Befalls mit Bakterien und Pilzen zu befürchten ist, da dies durch das eingeblasene Ozon als Oxidationsmittel wirksam verhindert wird. Demnach erweist sich das erfindungsgemäß Verfahren zur Vermeidung des mikrobiellen Befalls der Reinigungsvorrichtung X2 als äußerst wirksam. Zudem ist die Gefahr einer Kontamination anderer Pumpenköpfe X6 durch die Reinigungsvorrichtung X2 minimiert, auch wenn diese eine mikrobiell befallenen Pumpenkopf X6 reinigt - und sich damit entsprechende Verunreinigungen auf der zylindrischen Bürstenfläche X31 und im Reinigungsbad X34 sammeln - und im Anschluss daran weitere Pumpenköpfe X6 gereinigt werden.

Die in den nachfolgenden Punkten beschriebenen Gegenstände stellen weitere Ausführungsformen der Erfindung dar:
1. Verfahren zur Vermeidung des mikrobiellen Befalls einer Reinigungsvorrichtung (X2), insbesondere einer Reinigungsvorrichtung (X2) für eine Dosieranlage, wobei die Reinigungsvorrichtung (X2) wenigstens ein mechanisches Reinigungselement (X29) mit wenigstens einer Reinigungsfläche (X31) aufweist, dadurch gekennzeichnet, dass
   die wenigstens eine Reinigungsfläche (X31) des wenigstens einen mechanischen Reinigungselements (X29) zumindest zeitweise mit einem Oxidationsmittel beaufschlagt wird.
2. Verfahren nach Punkt 1,
   dadurch gekennzeichnet, dass
   das Oxidationsmittel auf die wenigstens eine mechanische Reinigungsfläche (X31) aufgesprüht oder aufgeblasen wird.
3. Verfahren nach Punkt 1 oder 2,
   dadurch gekennzeichnet, dass
   das Aufsprühen oder Aufblasen des Oxidationsmittels auf die wenigstens eine Reinigungsfläche (X31) im Wesentlichen entlang ihrer Längserstreckung, bevorzugt vollflächig erfolgt.
4. Verfahren nach einem der Punkte 1 bis 3,
   dadurch gekennzeichnet, dass
   das Oxidationsmittel ein Oxidationsfluid ist, insbesondere gasförmiges Oxidationsfluid, ist.
5. Verfahren nach Punkt 4,
   dadurch gekennzeichnet, dass
   das Oxidationsfluid Ozon enthält.
6. Verfahren nach einem der Punkte 1 bis 5,
   dadurch gekennzeichnet, dass
   die wenigstens eine Reinigungsfläche (X31) des wenigstens einen mechanischen Reinigungselements (X29) im Betrieb der Reinigungsvorrichtung (X2) zumindest zeitweise in einem Reinigungsbad (X34) gereinigt wird, wobei optional auch das Reinigungsbad (X34) mit dem Oxidationsmittel zumindest zeitweise beaufschlagt wird.
7. Verfahren nach Punkt 6,
   dadurch gekennzeichnet, dass
   das Aufsprühen oder Aufblasen des Oxidationsmittels auf die wenigstens eine Reinigungsfläche (X31) des wenigstens einen mechanischen Reinigungselements (X29) außerhalb des Reinigungsbads (X34) erfolgt.
8. Verfahren nach einem der Punkte 1 bis 7,
   dadurch gekennzeichnet, dass
   das wenigstens eine mechanische Reinigungselement (X2) eine umlaufende Reinigungsfläche (X31) aufweist.
9. Verfahren nach einem der Punkte 1 bis 8,
   dadurch gekennzeichnet, dass
   das wenigstens eine mechanische Reinigungselement (X29) zylindrisch oder walzenförmig ausgebildet ist.
10. Verfahren nach einem der Punkte 1 bis 9,
   dadurch gekennzeichnet, dass
   das wenigstens eine mechanische Reinigungselement (X29) wenigstens eine Bürste umfasst.
11. Verfahren nach Punkt 10,
   dadurch gekennzeichnet, dass
   das wenigstens eine mechanische Reinigungselement (X29) als rotierende zylindrische Bürste ausgebildet ist.
12. Verfahren nach Punkt 10 oder 11,
   dadurch gekennzeichnet, dass
   das Oxidationsmittel im Wesentlichen parallel zu den Borsten in die Bürste eingesprüht oder eingeblasen wird.
13. Verfahren nach einem der Punkte 1 bis 12,
   dadurch gekennzeichnet, dass
   das Oxidationsmittel über wenigstens eine Zuführleitung (X24, X26) zugeführt wird, wobei die wenigstens eine Zuführleitung (X24, X26) im Wesentlichen entlang der Längserstreckung der wenigstens einen Reinigungsfläche (X31) des wenigstens einen mechanischen Reinigungselements (X29) angeordnet ist, wobei die wenigstens eine Zuführleitung (X24, X26) eine, bevorzugt eine Mehrzahl, von auf die wenigstens eine Reinigungsfläche (X31) ausgerichtete Auslassöffnungen (X28) zum Aufsprühen oder Aufblasen des Oxidationsmittels aufweist.
14. Verfahren zum Reinigen einer Dosieranlage, insbesondere für eine Dispersion, speziell einer Farbdosieranlage, wobei die Dosieranlage wenigstens eine Dosiereinheit (X1) mit einem Pumpenkopf (X6) aufweist, wobei der Pumpenkopf (X6) mittels einer Reinigungsvorrichtung (X2) mit wenigstens einem wenigstens eine Reinigungsfläche (X31) aufweisenden mechanischen Reinigungselement (X29) gereinigt wird,
   dadurch gekennzeichnet, dass
   ein mikrobieller Befall der Reinigungsvorrichtung (X2) mit einem Verfahren nach einem der Punkte 1 bis 13 vermieden wird.
15. Reinigungsvorrichtung (X2) für eine Dosieranlage, insbesondere für eine Dispersion, speziell für eine Farbdosieranlage, umfassend wenigstens ein mechanisches Reinigungselement (X29) mit wenigstens einer Reinigungsfläche (X31) sowie eine Zuführeinheit (X24, X26) für ein Oxidationsmittel zur zumindest zeitweisen Beaufschlagung der wenigstens einen Reinigungsfläche (X31) mit einem Oxidationsmittel, wobei die Reinigungsvorrichtung (X2) konfiguriert ist zum Reinigen der Dosieranlage gemäß Punkt 14.
16. Reinigungsvorrichtung nach Punkt 15,
   dadurch gekennzeichnet, dass
   die Reinigungsvorrichtung (X2) ein Reinigungsbad (X34) zum zumindest zeitweisen Reinigen der wenigstens einen Reinigungsfläche (X31) des wenigstens einen mechanischen Reinigungselements (X29) im Betrieb der Reinigungsvorrichtung (X2) umfasst.
17. Dosieranlage für eine Dispersion, insbesondere Farbdosieranlage, umfassend eine Reinigungsvorrichtung (X2) nach Punkt 15 oder 16.

## Patentansprüche

1. Verfahren zur Vermeidung des mikrobiellen Befalls einer Reinigungsvorrichtung (X2), insbesondere einer Reinigungsvorrichtung (X2) für eine Dosieranlage, wobei die Reinigungsvorrichtung (X2) wenigstens ein mechanisches Reinigungselement (X29) mit wenigstens einer Reinigungsfläche (X31) aufweist, **dadurch gekennzeichnet, dass**
die wenigstens eine Reinigungsfläche (X31) des wenigstens einen mechanischen Reinigungselements (X29) zumindest zeitweise mit einem Oxidationsmittel beaufschlagt wird.

2. Verfahren nach Anspruch 1,
**dadurch gekennzeichnet, dass**
das Oxidationsmittel auf die wenigstens eine mechanische Reinigungsfläche (X31) aufgesprüht oder aufgeblasen wird, wobei das Aufsprühen oder Aufblasen des Oxidationsmittels auf die wenigstens eine Reinigungsfläche (X31) optional im Wesentlichen entlang ihrer Längserstreckung, bevorzugt vollflächig erfolgt.

3. Verfahren nach Anspruch 1 oder 2,
**dadurch gekennzeichnet, dass**
das Oxidationsmittel ein Oxidationsfluid ist, insbesondere gasförmiges Oxidationsfluid, ist und das Oxidationsfluid optional Ozon enthält.

4. Verfahren nach einem der Ansprüche 1 bis 3,
**dadurch gekennzeichnet, dass**
die wenigstens eine Reinigungsfläche (X31) des wenigstens einen mechanischen Reinigungselements (X29) im Betrieb der Reinigungsvorrichtung (X2) zumindest zeitweise in einem Reinigungsbad (X34) gereinigt wird, wobei optional auch das Reinigungsbad (X34) mit dem Oxidationsmittel zumindest zeitweise beaufschlagt wird.

5. Verfahren nach Anspruch 4,
**dadurch gekennzeichnet, dass**
das Aufsprühen oder Aufblasen des Oxidationsmittels auf die wenigstens eine Reinigungsfläche (X31) des wenigstens einen mechanischen Reinigungselements (X29) außerhalb des Reinigungsbads (X34) erfolgt.

6. Verfahren nach einem der Ansprüche 1 bis 5,
**dadurch gekennzeichnet, dass**
das wenigstens eine mechanische Reinigungselement (X2) eine umlaufende Reinigungsfläche (X31) aufweist.

7. Verfahren nach einem der Ansprüche 1 bis 6,
**dadurch gekennzeichnet, dass**
das wenigstens eine mechanische Reinigungselement (X29) zylindrisch oder walzenförmig ausgebildet ist.

8. Verfahren nach einem der Ansprüche 1 bis 7,
**dadurch gekennzeichnet, dass**
das wenigstens eine mechanische Reinigungselement (X29) wenigstens eine Bürste umfasst.

9. Verfahren nach Anspruch 8,
**dadurch gekennzeichnet, dass**
das wenigstens eine mechanische Reinigungselement (X29) als rotierende zylindrische Bürste ausgebildet ist.

10. Verfahren nach Anspruch 8 oder 9,
**dadurch gekennzeichnet, dass**
das Oxidationsmittel im Wesentlichen parallel zu den Borsten in die Bürste eingesprüht oder eingeblasen wird.

11. Verfahren nach einem der Ansprüche 1 bis 10,
**dadurch gekennzeichnet, dass**
das Oxidationsmittel über wenigstens eine Zuführleitung (X24, X26) zugeführt wird, wobei die wenigstens eine Zuführleitung (X24, X26) im Wesentlichen entlang der Längserstreckung der wenigstens einen Reinigungsfläche (X31) des wenigstens einen mechanischen Reinigungselements (X29) angeordnet ist, wobei die wenigstens eine Zuführleitung (X24, X26) eine, bevorzugt eine Mehrzahl, von auf die wenigstens eine Reinigungsfläche (X31) ausgerichtete Auslassöffnungen (X28) zum Aufsprühen oder Aufblasen des Oxidationsmittels aufweist.

12. Verfahren zum Reinigen einer Dosieranlage, insbesondere für eine Dispersion, speziell einer Farbdosieranlage, wobei die Dosieranlage wenigstens eine Dosiereinheit (X1) mit einem Pumpenkopf (X6) aufweist, wobei der Pumpenkopf (X6) mittels einer Reinigungsvorrichtung (X2) mit wenigstens einem wenigstens eine Reinigungsfläche (X31) aufweisenden mechanischen Reinigungselement (X29) gereinigt wird,
**dadurch gekennzeichnet, dass**
ein mikrobieller Befall der Reinigungsvorrichtung (X2) mit einem Verfahren nach einem der Ansprüche 1 bis 11 vermieden wird.

13. Reinigungsvorrichtung (X2) für eine Dosieranlage, insbesondere für eine Dispersion, speziell für eine Farbdosieranlage, umfassend wenigstens ein mechanisches Reinigungselement (X29) mit wenigstens einer Reinigungsfläche (X31) sowie eine Zuführeinheit (X24, X26) für ein Oxidationsmittel zur zumindest zeitweisen Beaufschlagung der wenigstens einen Reinigungsfläche (X31) mit einem Oxidationsmittel, wobei die Reinigungsvorrichtung (X2) konfiguriert ist zum Reinigen der Dosieranlage gemäß Anspruch 12.

14. Reinigungsvorrichtung nach Anspruch 13,
**dadurch gekennzeichnet, dass**
die Reinigungsvorrichtung (X2) ein Reinigungsbad (X34) zum zumindest zeitweisen Reinigen der wenigstens einen Reinigungsfläche (X31) des wenigstens einen mechanischen Reinigungselements (X29) im Betrieb der Reinigungsvorrichtung (X2) umfasst.

15. Dosieranlage für eine Dispersion, insbesondere Farbdosieranlage, umfassend eine Reinigungsvorrichtung (X2) nach Anspruch 13 oder 14.
